# EUROPEAN PATENT SPECIFICATION

(11) **EP 1 245 211 B1**
(45) Date of publication and mention of the grant of the patent: **10.05.2006**
(21) Application number: 02006716.1
(22) Date of filing: 22.03.2002
(51) Int. Cl.: A61F 13/534, A61F 13/15

(54) **Compound sanitary napkin**
Zusammengesetzte Damenbinde
Serviette hygiènique composée

(30) Priority: 30.03.2001 US 823044
(43) Date of publication of application: 02.10.2002
(73) Proprietor: McNEIL-PPC, INC., Skillman, NJ 08558 (US)
(72) Inventor: Glasgow, Tara, New Hope, PA 18938 (US); Rose, Kendra S., Chicago, IL 60614 (US); Mancuso, Michele, Sommerville, NJ 08876 (US)
(74) Representative: Groening, Hans Wilhelm

(56) References cited:
- EP-A- 0 916 328
- EP-A- 0 956 843
- WO-A-93/01783
- US-A- 4 608 047
- US-A- 6 042 575
- PATENT ABSTRACTS OF JAPAN vol. 1999, no. 09, 30 July 1999 (1999-07-30) & JP 11 104168 A (KAO CORP), 20 April 1999 (1999-04-20)

## Description

### FIELD OF THE INVENTION

The present invention relates to compound sanitary napkins that are designed and adapted to be worn in a crotch portion of a wearer's undergarment and to receive and contain menses and other vaginal discharges.

### BACKGROUND OF THE INVENTION

Disposable sanitary napkins generally comprise an absorbent element interposed between a liquid pervious body-faceable topsheet (often referred to as a cover layer) and a garment-faceable liquid impervious protective back sheet (often referred to as a barrier layer). The absorbent element is capable of receiving and containing liquids such as menses and other vaginal liquid discharges. The body faceable topsheet layer is intended to provide a comfortable and dry-feeling when in contact with a user's body while allowing free passage of fluid therethrough into the absorbent element. The protective back sheet layer is intended to retain the absorbed fluids within the sanitary napkin and thereby prevent the absorbed liquids from soiling the user's garments. Disposable sanitary napkins are generally provided with an adhesive attachment means for securing the article to a crotch portion of the user's undergarments. Sanitary napkins may also be provided with flexible side flaps along the longitudinal sides of the central absorbent element, which are adapted to be folded over the edges of a crotch portion of the user's undergarment.

Disposable sanitary absorbent products generally come in one of three basic configurations based upon their intended use. A first product configuration, which intended for heavy menstrual flow, is constructed with a relatively thick central absorbent element having a relatively high absorptive capacity. A second product configuration, which is intended for non-menstrual flow, has a thin flexible structure and is commonly referred to as a panty-liner. A third type of product configuration, which is also intended for heavy menstrual flow, has a thin, flexible structure and a relatively high absorptive capacity. Generally, the relatively high absorptive capacity is achieved by providing the thin absorbent element with superabsorbent particles. These sanitary napkins are commonly referred to as ultra-thin sanitary napkins.

Another class of sanitary napkins has recently been developed that combines several of these concepts into a single compound sanitary napkin. A compound sanitary napkin has a primary menstrual pad and a secondary undergarment protector that are joined to one another. Compound sanitary napkins of this design have been disclosed in U.S. Pat. No. 4,425,130 to DesMarais and Statutory Invention Registration H1614 to Mayer et al. In accordance with these references, the primary menstrual pad is intended to absorb the bulk of the bodily fluids discharged by the user, while the undergarment protector is intended to protect the user's garments from soiling. In use, the primary menstrual pad is adjacent the user's crotch region while the undergarment protector remains associated with the user's undergarment. The primary menstrual pad may be constructed to have a narrow width such that the pad at least partially resides within the external genitalia, or alternatively, may be wider than the distance between the labia majora. As the primary menstrual pad is made narrower to fit the body, the secondary menstrual pad preferably remains sufficiently wide enough to provide a stable attachment to the wearer's undergarment and to sufficiently cover the undergarment to protect it from soiling. In either embodiment, the primary menstrual pad is generally adapted to be laterally compressible and conformable under relatively low forces. Thus in use, the forces exerted by the soft tissue of the female external genitalia compress the primary menstrual pad such that a portion of the primary menstrual pad is able to at least partially reside within the external female genitalia. By being conformable at relatively low forces, the primary absorbent member remains comfortable during use. In addition, the primary menstrual pad preferably exhibits a resilient recovery to enable the pad to conform to the body as the pad and body interface is subjected to shape changes.

In EP 0 956 843 A1 a sanitary napkin is disclosed having a first member and a second member placed upon the first member. The first member comprises a liquid-pervious topsheet, a liquid impervious backsheet and a first liquid absorbent core disposed between the topsheet and the backsheet. Portions of the topsheet and the backsheet extend outward beyond a peripheral edge of the first core and are placed upon and bonded to each other by means of a suitable adhesive agent or heat-sealing technique. The second member comprises liquid-pervious and elastic sheets and a second liquid absorbent core disposed between these two sheets. Portions of these liquid pervious and elastic sheets extend outward beyond a peripheral edge of the second core and are placed upon and bonded to each other by means of a suitable adhesive agent or heat-sealing technique. The first member and the second member have longitudinally opposite end portions and transversely opposite side edge portions. The transversely opposite side edge portions extend longitudinally along transversely opposite side edge portions of a crotch region of the undergarment. The longitudinally opposite end portion of the second member are joined to an upper surface of the topsheet of the first member over its portions extending outward beyond a peripheral edge of the first core. When the second member is placed under longitudinal tension, its longitudinally opposite end portions are joined to longitudinally opposite end portions of the first member so that the second member causes the first member to be longitudinally curved downward as the second member elastically contracts. With the sanitary napkins of EP 0 956 843 Al the leakage of body exudates due to misplacement of the napkin shall be eliminated.

### SUMMARY OF THE INVENTION

It is an object of the present invention to provide a compound sanitary napkin that provides enhanced fit to a wearer's body.

In accordance with the present invention there has been provided a compound sanitary napkin that is capable of dynamically fitting a wearer in use comprising an uppermost primary absorbent member and a lowermost secondary absorbent member, which are joined to each other to form a unitary structure. The primary absorbent member includes an absorbent core and a fluid pervious topsheet superposed on the absorbent core and has a first transverse end and an opposite second transverse end and a first longitudinal side and an opposite second longitudinal side defining therebetween a width. The secondary absorbent member includes a fluid pervious topsheet and a fluid impervious barrier sheet joined to at least a portion of the topsheet and has a first transverse end and an opposite second transverse end and a first longitudinal side and an opposite second longitudinal side defining therebetween a width. At least a portion of the primary absorbent member along or adjacent to its first transverse end is joined to at least a portion of the secondary absorbent member along or adjacent to its first transverse end at a first union means and at least a portion of the primary absorbent member along or adjacent to its second transverse end is joined to at least a portion of the secondary absorbent member adjacent along or adjacent to its second transverse end at a second union means. The primary absorbent member has a center portion intermediate the first and second union means and the secondary absorbent member has a central region intermediate the first and second union means, the center portion and the central region being unaffixed to each other and each having a respective length measured from the first union means to the second union means. The center portion of the primary absorbent member has a length that is less than the length of the central region of the secondary absorbent member. Thus, the length of the central region of the secondary absorbent member is foreshortened and at least a portion of the center portion of the primary absorbent member is spaced apart from the central region of the secondary absorbent member to provide a dynamic fit to the wearer.

### BRIEF DESCRIPTION OF THE DRAWINGS

While the specification concludes with claims particularly pointing out and distinctly claiming the present invention, it is believed that the present invention will be better understood from the following description in conjunction with the following drawings, in which like reference numbers identify identical elements and wherein:
FIG. 1 is a top plan view of one embodiment of the compound sanitary napkin not according to the present invention.
FIG. 2 is a side elevation view of the compound sanitary napkin shown in FIG. 1 as taken along section lines 2--2 of FIG 1.
FIG. 3 is a side elevation view of the compound sanitary napkin shown in FIG. 1 as taken along section lines 3--3 of FIG 1.
FIG. 4 is a side elevation view of another embodiment of the compound sanitary napkin not according to the invention.
FIG. 5 is a side elevation view of another embodiment of the compound sanitary napkin not according to the invention.
FIG. 6 is a side elevation view of the compound sanitary napkin shown in FIG. 5 as taken along section lines 6--6 of FIG 5.
FIG. 7 is a side elevation view of the a transverse end region of an embodiment of a compound sanitary napkin of the present invention.
FIG. 8 is a side elevation view of another embodiment of a compound sanitary napkin not according to the present invention.
FIG. 9 is a side elevation view of another embodiment of a compound sanitary napkin not according to the present invention.
FIG 10 is a side elevation view of the compound sanitary napkin shown in FIG 9 in an in-use position.
FIG. 11 is a top plan view of another embodiment of a compound sanitary napkin of the present invention
FIG. 12 is a top plan view of another embodiment of a compound sanitary napkin of the present invention.

### DETAILED DESCRIPTION OF THE INVENTION

The present invention is directed to a compound sanitary napkin that is adapted to dynamically fit a wearer in use to provide enhanced body fit and thereby better enable the napkin to absorb body fluids and to protect a user's garments from being soiled. The term "sanitary napkin", as used herein, refers to a disposable absorbent article such as panty liners, sanitary napkins, adult incontinence devices and the like, which are worn by females in a crotch portion of an undergarment adjacent to the pudendal region and which are intended to absorb and retain the various liquid exudates which are discharged from the body (e.g., blood, menses, and urine). As used herein, the term "pudendal" refers to the externally visible female genitalia and is limited to the labia majora, the labia minora, the clitoris, and the vestibule. The term "compound sanitary napkin", as used herein, refers to a sanitary napkin comprised of a primary absorbent member and a secondary absorbent member which are separate constituents joined to one another to form a unitary structure. The primary absorbent member is adapted to contact the body of the user and is intended to absorb the bulk of bodily fluids discharged by the user. The secondary absorbent member is adapted to protect the user's garments from soiling by absorbed fluids which may be expelled from the primary absorbent member or which may inadvertently bypass the primary absorbent member.

The compound sanitary napkin has a longitudinal centerline and a transverse centerline. The term "longitudinal", as used herein, refers to a line, axis or direction which lies within the plane of the compound sanitary napkin that is generally parallel to a vertical plane which bisects a standing wearer into left and right body halves when the compound sanitary napkin is worn. The term "lateral", as used herein refers to a line, axis, or direction which lies within the plane of the compound sanitary napkin that is generally perpendicular to the longitudinal direction. The compound sanitary napkin has an upper body faceable surface and a lower garment faceable surface. The primary and secondary absorbent members each have respective longitudinal centerlines, transverse centerlines, first and second opposite transverse ends, body faceable surfaces and garment faceable surfaces.

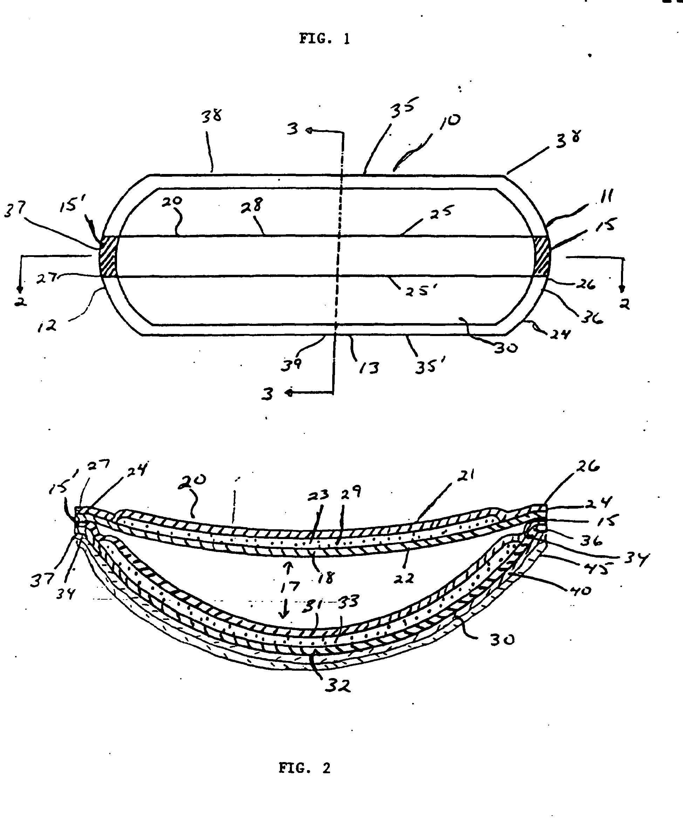

Referring now to the Figures in which like reference numbers identify identical elements, and in particular to Figures 1-3 which illustrate a sanitary napkin 10 having a primary absorbent member 20 and a secondary absorbent member 30 each having respective opposite transverse ends. The compound sanitary napkin is formed by affixing at least a portion of the primary absorbent member 20 along or adjacent to its first transverse end 26 to a portion of the secondary absorbent member 30 along or adjacent to at least a portion of its first end region 36 with a first union means 15 and affixing at least a portion of the primary absorbent member 20 along or adjacent to its second transverse end 27 to at least a portion of the secondary absorbent member 30 along or adjacent to its second end region 37 with a second union means 15'. The first and second union means 15, 15' are located solely in the respective transverse end regions of the napkin and thus do not extend along the entire length of the compound sanitary napkin 10. Thus, a central region 39 of the secondary absorbent member 30 and a center portion 18 of the primary absorbent member remain unaffixed to each other.

It is considered an important feature of the present invention that the unaffixed center portion 18 of the primary absorbent member 20 has a length that is less than the length of the unaffixed central region 39 of the secondary absorbent member 30. The length of the center portion 18 is measured from the first union means 15 to the opposite second union means 15'. Similarly, the length of the central region 39 of the secondary absorbent member 30 is measured from the first union means 15 to the opposite second union means 15'. Since the primary absorbent member 20 is affixed to the secondary absorbent member 30 at the first and second union means 15, 15', it causes the length of the central region 39 of the secondary absorbent member 30 to become foreshortened. As used herein, the terminology "foreshortened" refers to a decrease in the linear length of the secondary absorbent member relative to its original linear length (i.e. its length prior to its affixation of the primary absorbent member) due to the curvature imparted by the primary absorbent member. That is, upon affixation of the primary absorbent member 20 to the secondary absorbent member 30, the shape of the secondary absorbent member becomes slightly bowed or curved away from the primary absorbent member. The curved configuration imparted to the secondary absorbent member by the primary absorbent member also creates a z-directional gap 17 between the planes defined by the primary absorbent member and the secondary absorbent member. Thus, at least a portion of the center portion 18 of the primary absorbent member 20 is vertically spaced apart from the central region 39 of the secondary absorbent member 30. Controlling the relative difference in lengths of the respective center portion 18 and central region 39 of the absorbent members will control the amount of gap 17. The gap 17 is generally between 1 mm and 40 mm and is preferably between 2 mm and 30 mm and most preferably between 3 mm and 20 mm. In general, the center portion 18 of the primary absorbent member has a length that is between 10% to about 98% of the length of the central region 39 of the secondary absorbent member. The length of the center portion 18 of the primary absorbent is preferably less than 98% of the length of the central region 39 of the secondary absorbent member, more preferably between 95 % to about 50% and most preferably between 95% and 75% of the length of the central region 39 of the secondary absorbent member 30.

Since the central region 39 and the center portion 18 of the compound sanitary napkin are unaffixed to each other, the center portion 18 of the primary absorbent member 20 is capable of moving independently of-the central region 39 of the secondary absorbent member 30. The length of the unattached center portion 18 between the union means generally controls the amount of movement of the primary absorbent member relative to the secondary absorbent member. Thus, if more or less movement is desired, the length of the unattached center portion 18 may be adjusted accordingly. The length of the unattached center portion 18 will generally extend from about 99% to about 25% of the length of the primary absorbent member and is preferably from about 95% to about 50% of the length of the primary absorbent member. Thus, the combined length of each respective union means 15, 15' will generally extend from about 1% to about 75% of the length of the primary absorbent member. Preferably the union means extends from about 3% to about 50%, more preferably from about 5% to about 30% of the length of the primary absorbent member. The length of each respective union means 15, 15' in each end of the compound sanitary napkin may be the same or different. The transverse ends of the primary absorbent member are shown as being substantially coterminous with and joined to the transverse ends of the secondary absorbent member along union means 15. However, one or both transverse ends 26, 27 of the primary absorbent member may extend beyond or be joined inward (as illustrated in Figure 16) of the respective first and second ends 36, 37 of the secondary absorbent member 30 provided of course that the central region 39 remains unaffixed.

The precise nature of the union means 15, 15' is not, per se, critical to the invention, provided of course that the union means selected serves to join the primary absorbent member 20 and the secondary absorbent member 30 with sufficient tenacity that they do not become separated during use and thus the compound sanitary napkin remains a unitary structure during use. Union means such as adhesive attachment with well known hot melt adhesives and pressure sensitive adhesives have been found to provide a satisfactory means for joining the primary absorbent member to the secondary absorbent member. If the nature of the components selected to construct the constituents of the compound sanitary napkin so permit, heat welding, ultrasonic welding, or a combination of both heat and ultrasonic welding can be used.

In accordance with the embodiment shown in Figures 1-3, each respective transverse end 26, 27 of the primary absorbent element 20 is formed from continuous extensions of the topsheet 21 and back sheet 22 that extend beyond the peripheral edge margins of the absorbent means 23 and are sealed together to form flange 24. Similarly, each respective transverse end 36, 37 of the secondary absorbent element 30 is formed from a continuous extension of the peripheral edge margins of the topsheet 31 and back sheet 32 that extend beyond the peripheral edges of the absorbent element 33 (if any) and are sealed together to form flange 34. The union means 15, 15' are located in the each of the respective flange portions of the transverse ends of the primary absorbent member and secondary absorbent member and unite the component parts into a unitary structure.

The primary absorbent member 20 has opposite longitudinally extending sides 25, 25' defining therebetween a width, a first transverse end 26 and an opposite second transverse end 27 defining therebetween a length. The longitudinally extending sides 25, 25' and the transverse ends 26, 27 together define the periphery 28 of the primary absorbent member. The secondary absorbent member 30 has opposite longitudinally extending sides 35, 35' defining therebetween a width and opposite laterally extending transverse ends 36, 37 defining therebetween a length and which together form the periphery 38 of the secondary absorbent member. In accordance with a preferred embodiment, the primary absorbent member has a length that is preferably less than the length of the secondary absorbent member. The width of the primary absorbent member is generally equal to or less than the width of the secondary absorbent member. Thus, in accordance with this embodiment, the periphery 38 of the secondary absorbent member 30 also defines the periphery of the compound sanitary napkin 10. The primary absorbent member 20 includes an absorbent means 23 and a liquid permeable topsheet 21 superimposed on the absorbent means 23.

The topsheet 21 may be any liquid pervious material that is capable of permitting liquid to readily penetrate through its thickness and is preferably compliant, soft feeling, and non-irritating to the wearer's skin. A suitable topsheet 21 may be manufactured from a wide range of materials such as woven fabrics, nonwoven fabrics, apertured polymeric films, porous foams, apertured foams, reticulated films and the like and combinations thereof. Suitable woven fabrics and nonwoven fabrics can be comprised of natural fibers (e.g., wood or cotton fibers), synthetic fibers (e.g., polymeric fibers such as polyester, polypropylene, or polyethylene fibers) or from a combination of natural and synthetic fibers. A preferred topsheet comprises a synthetic nonwoven fabric formed from a homogeneous mixture of 3 denier polypropylene fibers and 5 denier polypropylene fibers. Another preferred topsheet comprises a three dimensional apertured film. Particularly preferred apertured films are pervious to body exudates and yet non-absorbent and have a reduced tendency to allow liquids to pass back through and rewet the wearer's skin. Thus, the surface of the film that is in contact with the body remains dry, thereby reducing body soiling and creating a more comfortable feel for the wearer. The body-faceable or exposed surface of the film topsheet may be treated to make it hydrophilic and thus help liquid transfer through the topsheet faster. This has been found to diminish the likelihood that menstrual fluid will flow off the topsheet rather than flowing into and being absorbed by the absorbent core. In a preferred embodiment, surfactant is incorporated into the polymeric materials of the topsheet. Alternatively, the body surface of the topsheet can be made hydrophilic by treating it with a surfactant.

To insure proper fluid transfer between the topsheet 21 and the subjacent absorbent means 23 it is preferred that the topsheet 21 be at least partially secured to the absorbent means 23 throughout their common association or interface. By securing at least a portion of the topsheet 21 to the underlying absorbent means 23, the topsheet 21 will have a reduced tendency to separate from the absorbent means 23 during use which may inhibit fluid transfer from the top sheet 21 into the underlying absorbent means. The topsheet 21 may be secured to the absorbent means 23 in any suitable manner including, but not limited to spray gluing or applying lines or spots of adhesives between the topsheet 21 and the absorbent means. Alternatively, or additionally, the topsheet 21 may be secured to the absorbent means 23 by entangling the fibers of the absorbent means with the topsheet 21, by fusing the topsheet to the absorbent means 23 with a plurality of discrete individual fusion bonds.

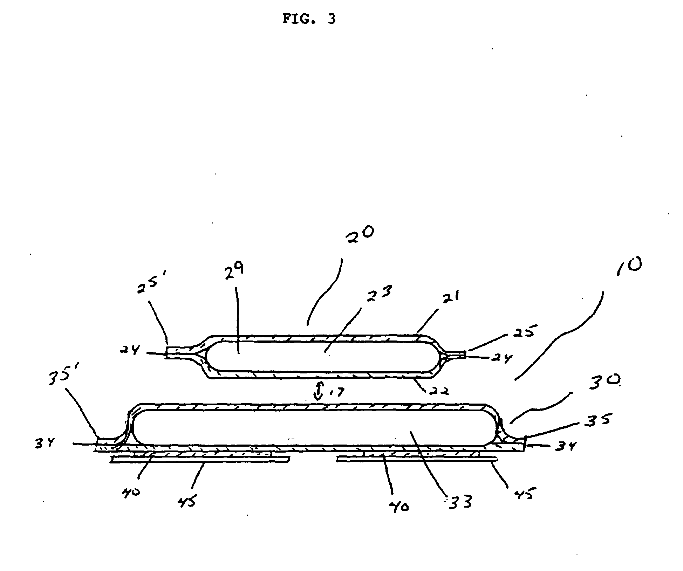

The absorbent means 23 may be formed from any absorbent material that is generally non-irritating to the wearer's skin and capable of absorbing and containing body exudates. Suitable materials for the absorbent means are preferably compliant, soft, comfortable and resilient to enhance body fit and comfort of the primary absorbent member. Preferably, the absorbent means is compressible so that the primary absorbent member will deform under relatively small forces that may be experienced during normal use. In addition to being compressible, the materials comprising the absorbent means are preferably conformable such that the primary absorbent member is able to provide improved fit into and around the labia and perineum. While being generally compressible and conformable under relatively small forces, those forces exerted by the external female genitalia during use, it is also preferred that the primary absorbent member be sufficiently resilient such that when subjected to normal wearing forces it does not permanently collapse. Preferably, the primary absorbent member will be sufficiently resilient that it will conform to the contours of the body to provide intimate contact with the exposed genitalia of the female user. Intimate contact with the exposed female genitalia helps provide better fluid transfer from the user into the primary absorbent member without allowing fluid to bypass and/or run-off the primary absorbent member. While the resilient characteristics of the absorbent means allow for improved fit, they must be balanced against the need for the product to be both soft and comfortable for the wearer. The absorbent means 23 may include a wide variety of liquid absorbent materials commonly used in disposable sanitary napkins, and other disposable absorbent articles. Examples of suitable absorbent materials include defiberized wood pulp (which is generally referred to as pulp fluff), creped cellulose wadding, modified cross-linked cellulose fibers, capillary channel fibers (that is, fibers having intra-fiber capillary channels), absorbent foams, thermally or adhesively bonded airlaid materials, absorbent sponges, synthetic staple fibers, polymeric fibers, hydrogel-forming polymer gelling agents (commonly referred to as superabsorbent particles or fibers), peat moss, or any equivalent materials or combinations of materials. In the embodiment illustrated in FIG. 3, the absorbent means includes only a single layer absorbent core 29. In a preferred embodiment, absorbent core 29 is comprised of defiberized pulp fibers. The absorbent core 29 has a generally oval shape to provide the primary absorbent member 20 with a generally oval shape. While the absorbent means 23 shown in FIGS. 3 has a generally oval cross-section, the absorbent core may be manufactured in a wide variety of shapes wherein the cross-section is a rectangle, triangle, oval, square, U-shape, Z-fold, etc. As shown in FIG. 1, the primary absorbent member has a generally rectangular shape. However, other suitable shapes include but are not limited to oval, hourglass, dog-bone, asymmetric, etc.

Absorbent means 23 may further comprise a fluid acquisition layer (not shown) positioned between the topsheet 21 and an absorbent core 29. The fluid acquisition layer may serve several functions including improving wicking of exudates over and into the absorbent core 29. By improving the wicking of exudates, the acquisition layer provides a more even distribution of the exudates throughout the absorbent core. The fluid acquisition layer may be comprised of several different materials including nonwoven or woven webs of synthetic fibers including polyester, polypropylene, or polyethylene; natural fibers including cotton or cellulose; blends of such fibers; or any equivalent materials or combinations of materials. In a preferred embodiment, the acquisition layer may be joined with the topsheet by any of the conventional means for joining webs together such as for example using adhesive, thermo-bonding techniques, and the like.

The primary absorbent member 20 is preferably relatively thin (i.e. having a caliper less than 5 millimeters.) and flexible. Preferably, the primary absorbent member will have a caliper of less than about 3.0 millimeters, more preferably less than about 2.6 millimeters, more preferably less than about 2.2 millimeters, and most preferably less than about 2.0 millimeters. It may be desirable to provide a primary absorbent member with varying degrees of caliper throughout its length. For example, the primary absorbent member may be relatively thicker in the central region and taper towards the end regions. Alternatively, the primary absorbent member may be relatively thinner in the central region relative to the end regions. The width of the primary absorbent member can vary widely depending on the desired level of comfort or fit desired and may optionally vary along its length. For example, the primary absorbent member may be adapted to reside partially within and partially external of the wearer's vestibule and/or gluteal groove. In accordance with this embodiment, the primary absorbent member has a width of about 10 mm to comfortably fit within at least a portion of the labial groove and/or gluteal groove. However, primary absorbent members have widths greater than 10 mm may also be adapted to fit within the labial groove provided of course that it is relatively conformable so that it will readily fit into the labial groove during use. In a preferred embodiment, the width of the primary absorbent member 20 is less than the width of a user's groin and is at least the width of a user's labia majora. The width of the primary absorbent member 20 in the center portion 18 will generally range from about 10 mm to 65 mm, preferably from about 10 mm to about 40 mm. The width of the primary absorbent member at one or both of its transverse ends 26, 27 may be the same as the width of the center portion 18 or may be greater than or less than the width of the center portion 18. In general, the width of the transverse ends will range from about 10 mm to 85 mm.

The width of the primary absorbent member 20 may also be varied depending upon the caliper (i.e. thickness) of the and the width of the compound sanitary napkin 10. If the combined calipers of the primary absorbent element 20 and the secondary absorbent member 30 in a center region 13 of the napkin result in a thick, bulky sanitary napkin (i.e. greater than 5 mm), the width of the secondary absorbent member may be relatively wide, e.g. 65-75 mm while the width of the primary absorbent member in the center portion 18 should be relatively narrow, generally less than 40 mm. Conversely, if the combined calipers of the primary absorbent element and the secondary absorbent member result in a thin or ultra-thin sanitary napkin (i.e. less than 5 mm), then the width of both the primary and secondary absorbent members may be relatively wide. In a preferred embodiment, a center portion 18 of the primary absorbent element 20 and the central region 39 of the secondary absorbent member 30 have a combined thickness of less than 5 mm, the width of the primary absorbent member ranges from about 10 mm to about 40 mm and the width of the secondary absorbent member ranges from about 50 mm to 75 mm.

The length of the primary absorbent member 20 can be of any convenient dimension and is generally from about 5 cm to about 40 cm, more preferably from about 10 cm to 35 cm, and most preferably from about 15 cm to 30 cm. A particularly preferred primary absorbent member 20 has a length of about 25 cm.

The total absorbent capacity of the absorbent means 23 should be compatible with the intended exudate loading for the compound sanitary napkin 10. Further, the absorbent capacity of the absorbent means 23 may be varied to accommodate wearers ranging in the expected amount of exudate fluid volume. For instance, a different absorbent capacity may be utilized for a compound sanitary napkin intended for daytime use as compared with one intended for nighttime use, or for a compound sanitary napkins intended for use by teenage females as compared with one intended for use by more mature women. The total absorbent capacity is preferably greater than about 14 g of fluid.

The secondary absorbent member 30 comprises a liquid pervious topsheet 31 and a liquid impervious barrier sheet 32. The topsheet 31 and the barrier sheet 32 are joined to one another along their respective peripheral edge margins. The topsheet 31 can be any fluid pervious material commonly used in sanitary napkins, disposable diapers, and the like and may be any of the materials described above as being useful in the topsheet 21 of the primary absorbent member 20. A preferred topsheet 31 comprises an apertured film. The barrier sheet 32 is impervious to liquids (e.g., menses and/or urine) and is preferably formed from a thin, flexible plastic film, although other flexible liquid impervious materials may also be used. In use, the barrier sheet 32 is interposed between the topsheet 31 and the user's undergarments. The function of the barrier sheet 32 is to prevent exudates which may be expelled from or which inadvertently bypass the primary absorbent element 20 and exudates contained within the secondary absorbent member 30 from contacting and soiling the user's undergarments. The barrier sheet 32 may thus comprise a woven or nonwoven material, polymeric films such as thermoplastic films of polyethylene or polypropylene, or composite materials such as a film-coated nonwoven material. Preferably, the barrier sheet is a polyethylene film having a thickness of from about 0.012 mm (0.5 mil) to about 0.015 mm (2.0 mil). Exemplary polyethylene films are manufactured by Clopay Plastic Products Co. having an address at 312 Walnut St Cincinnati, OH 45202, Huntsman Packaging having an address at 230 Enterprise Drive, Newport News, VA 23603, Tredegar Industries having an address at 1100 Boulders Parkway, Richmond, Virginia, Exxon Chemical Co. having an address at 750 West Lake Cook Road, Buffalo Grove, Illinois 60089. The barrier sheet is preferably embossed and/or matte finished to provide a more cloth-like appearance. Further, the barrier sheet may permit vapors to escape from the secondary absorbent member (i.e., breathable) while still preventing exudates from passing through the barrier sheet.

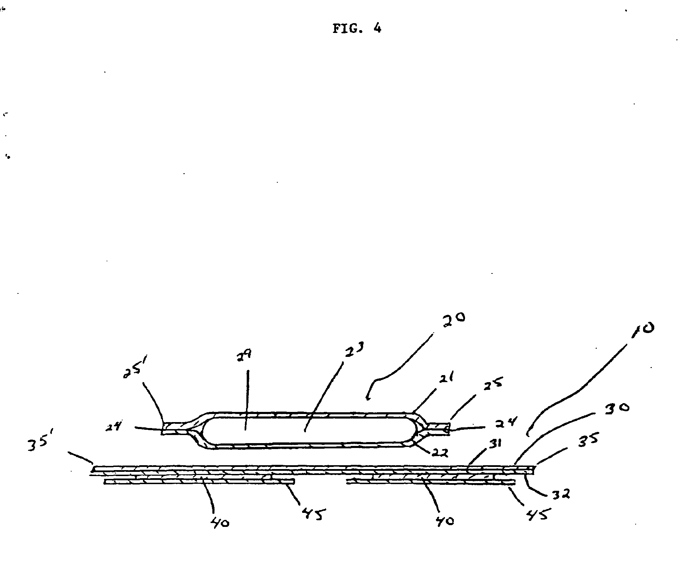

The embodiment shown in FIG 4 is similar in all aspects to the embodiments in FIGURES 1-3 except that the secondary absorbent member is formed solely from a body faceable topsheet 31 and a garment faceable barrier sheet 32. In accordance with this embodiment, there is no absorbent material intermediate the topsheet and barrier sheet as such is not required. As noted above, most if not all of the bodily fluids are preferably absorbed by and are contained within the absorbent core of the primary absorbent member. Thus, the secondary absorbent member may be adapted to protect the user's garments from soiling by relatively small amounts of fluids which may be expelled from the primary absorbent member or which may inadvertently bypass the primary absorbent member. Accordingly, since the expected amounts of fluids that may come into contact with the secondary absorbent are relatively small, an absorbent element may not be necessary to contain the fluids within the secondary absorbent member and prevent them from soiling the user's garments.

However, in accordance with an embodiment as shown in FIGS 1-3, the secondary absorbent member includes an absorbent element 33 between the topsheet 31 and the barrier sheet 32. The absorbent element 33 can be any absorbent material commonly used in sanitary napkins, disposable diapers, and the like and may be any of the materials described above as being useful in the absorbent means of the primary absorbent member. However, because the absorbent element 33 of the secondary absorbent member performs a different function from that of the absorbent means 23, the absorbent element 33 can be, and most preferably is, somewhat thinner and less bulky than the absorbent means 23. Accordingly, the absorbent element 33 can be formed of different materials than the absorbent means 23. For example, single or more plies of paper tissue (as commonly used in paper toweling or toilet tissue) can be used to form the absorbent element. Preferably, the absorbent element is formed from about 1 to about 5 plies of paper tissue. Paper tissue having one or more plies, a basis weight of from about 24 to about 48 grams per square meter and an apparent density of from about 0.10 to about 0.12 grams per cubic centimeter has been found provide satisfactory absorbent capacity for use as the absorbent element 33. Wet strength resins and latex binders are preferably used to provide additional strength to the paper tissue used in the absorbent element.

The length of the secondary absorbent member 30 can be of any convenient dimension and generally has a length of from about 20 cm to 40 cm, preferably from about 25 cm to 35 cm, and most preferably about 30 cm. The secondary absorbent member is preferably relatively thin and flexible. Preferably, the secondary absorbent member will have a caliper of less than about 3.0 millimeters, more preferably less than about 2.6 millimeters, more preferably less than about 2.2 millimeters, and most preferably less than about 2.0 millimeters. In a most preferred embodiment, the secondary absorbent member has a caliper of about 1.9 millimeters. While it can be of generally any cross-section in its unstressed condition, the secondary absorbent member is preferably from about 5 to 15 cm in width, more preferably from about 5 to 10 cm in width, and most preferably from about 5 to 8 cm in width. Preferably, the width of the secondary absorbent member is at least 1.5 times the width of the primary absorbent member. More preferably, the width of the secondary absorbent member is at least 2 times the width of the primary absorbent member. Most preferably, the width of the secondary absorbent member is in the range from about 3 to about 8 times the width of the primary absorbent member. As shown in FIG. 1, the secondary absorbent member has a generally rectangular shape. However, other suitable shapes include but are not limited to oval, hourglass, dog-bone, asymmetric, etc.

The secondary absorbent member 30 is preferably provided with an attachment means 40, such as an adhesive. The attachment means 40 provides a means for securing the compound sanitary napkin 10 into the crotch portion of the user's undergarment. Thus, at least a portion of the garment faceable surface of the barrier sheet 32 is coated with a pressure sensitive adhesive that is capable of releasably securing the compound sanitary napkin to an undergarment. In a preferred embodiment, at least a portion of the attachment means 40 is positioned on the garment faceable surface of the barrier sheet 32 adjacent the longitudinal side edges 35, 35' of the secondary absorbent member 30. Any adhesive or glue used in the art for such purposes can be used for the adhesive herein, with pressure-sensitive adhesives being preferred. Suitable adhesives include hot melt adhesives such as HL 1417XZP adhesive and HL 1491XZP adhesive which are commercially available from HB Fuller Corporation St. Paul, MN 55110 or H2262 adhesive and H2543 adhesive which are commercially available from ATO Findley Inc. Wauwatosa, WI 53226. The pressure-sensitive adhesive is typically covered with a removable release liner 45 in order to keep the adhesive from drying out or adhering to a surface other than the crotch portion of the undergarment prior to use. Any commercially available release liners commonly used for such purposes can be utilized herein. A suitable release liner is commercially available from Tekkote Corporation, Leonia, NJ 07605. The compound sanitary napkin 10 of the present invention is used by removing the release liner 45 and thereafter placing the sanitary napkin in an undergarment so that the adhesive contacts the undergarment. The adhesive is adapted to maintain the sanitary napkin in its position within the undergarment during use.

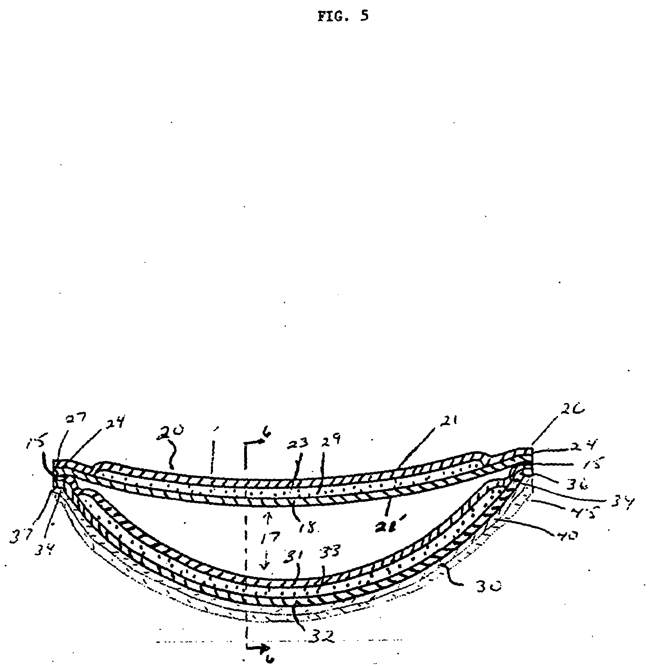

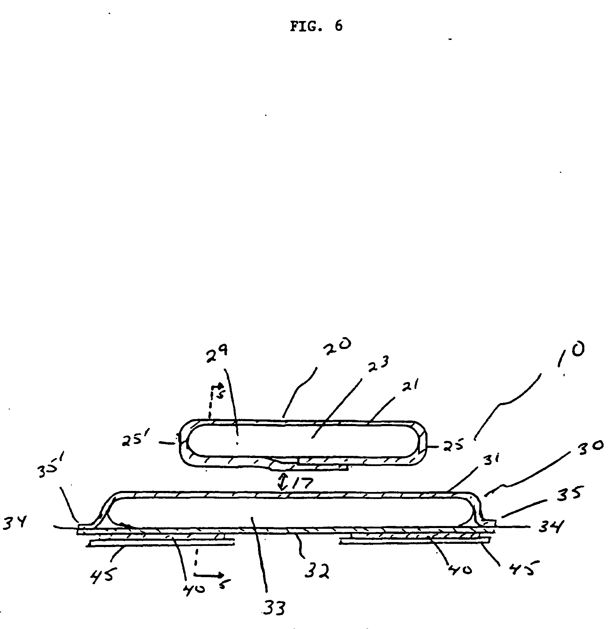

FIGS. 5 and 6 illustrate an embodiment in which like reference numbers identify identical elements as in the embodiment shown in FIGS 1-3. In accordance with this embodiment, the primary absorbent member 20 includes an absorbent means 23 and a liquid permeable topsheet 21, 21' superimposed on all sides of the absorbent means 23. Thus, the body faceable surface and the garment faceable surface of the primary absorbent member are formed from a single topsheet 21 to fully enclose the absorbent means 23. Each respective transverse end 26, 27 of the primary absorbent element 20 is formed from a continuous extension of the topsheet 21 that extends beyond the peripheral edge margins of the absorbent means 23 and is sealed together to form flange 24. Each respective transverse end 36, 37 of the secondary absorbent element 30 is formed from a continuous extension of the topsheet 31 and back sheet 32 that extend beyond the peripheral edge margins of the absorbent element 33 and sealed together to form flange 34. The union means 15, 15' are located in the each of the respective transverse ends of the primary absorbent member and secondary absorbent member and unite the component parts into a unitary structure.

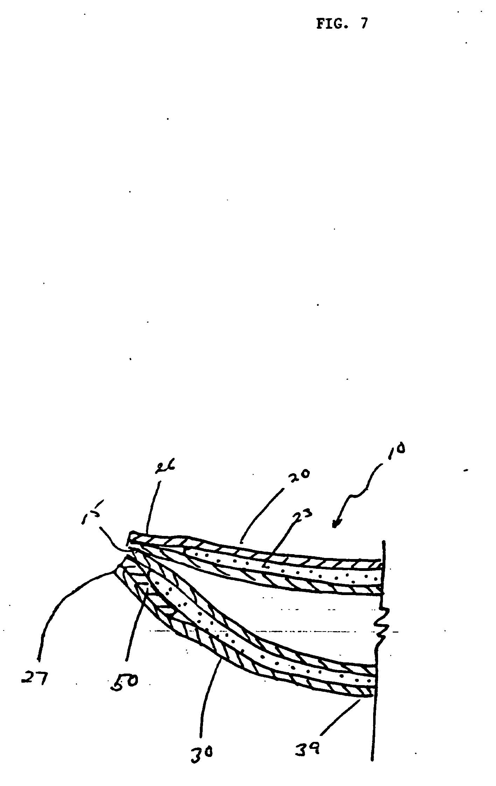

When a compound sanitary napkin of the present invention is placed in an undergarment and worn by a user, it is important that any compressive forces that may be applied to the primary absorbent member by the user's body not distort the transverse end regions of the secondary absorbent member causing these end regions to fold inward. Thus, it is in accordance with the present invention that the transverse end regions of the secondary absorbent member be sufficiently stable to maintain their intended shape in use (i.e. to conform to the shape of the wearer's undergarment) when subjected to stresses that may be imparted by the primary absorbent member. More particularly, when the compound sanitary napkin of the present invention is in use, the wearer's body may impart downward compressive forces to the primary absorbent member. These downward compressive forces will in turn impart an inwardly oriented force to the union means that may tend to distort the transverse ends of the secondary absorbent member by causing them to fold inward towards the center of the napkin. As shown if FIG 7, a means according to the invention to reduce or eliminate the distortion of the transverse ends of the secondary absorbent member is to include a stabilizing element 50 into the transverse end region 27 of the secondary absorbent member 30. The choice of a particular material or process to form a stabilizing element 50 is not, per se, critical to the invention, provided of course that it is capable of preventing the transverse ends of the compound napkin from folding inward towards the center of the napkin.

In a preferred embodiment, the stabilizing element 50, when subjected to an inwardly compressive force, creates a bending moment in the secondary absorbent member that is located at a position that is spaced apart from the union means. Preferably, the bending moment is located at a position that is intermediate the opposite union means in each respective transverse end region of the secondary absorbent member. That is, any inwardly oriented forces that may be imparted to the secondary absorbent member by the primary absorbent member 20 are preferably distributed over a portion of the secondary absorbent member 30 that extends inwardly beyond the union means 15. The bending moment is preferably located inward from the absorbent core 23 in the primary absorbent member 20, inward from the adhesive attachment means 40 or inward from the absorbent member 33 in the secondary absorbent member 30 (if any). The choice of particular material or method to provide a suitable stabilizing element 50 can vary widely, depending on the desired structure of the secondary absorbent member. For example, the stabilizing element 50 may comprise additional materials or processes that impart stiffness to the transverse end regions of the secondary absorbent member. The regions of increased stiffness preferably extend from the union means 15, 15' inward towards the central region 39 of the secondary absorbent element.

In accordance with another embodiment, the stabilizing member 30 includes an additional adhesive layer that is capable of imparting stiffness to the transverse end region 27 of the secondary absorbent member 30. Alternatively, one or more layers of an absorbent material (such as pulp fluff or one or more tissue layers) may be included in the transverse end region in an amount sufficient to create a bending moment that is inward from each respective union means of the secondary absorbent member 30. Combinations of these materials are also contemplated as being within the scope of the present invention. An example of another preferred process that may be used to impart stiffness to the end region is to crimp or emboss a channel to create a bending moment intermediate each of the opposite union means of the secondary absorbent member.

In accordance with the foregoing embodiments, the stabilizing element 50 has a first end that is substantially in vertical registration with at least a portion of the union means 15 and extends longitudinally inward towards the center region to an extent that a second opposite end of the stabilizing element 50 is substantially in vertical registration with at least a portion of the absorbent means 23 in the primary absorbent member 20. In a preferred embodiment, the stabilizing element 50 overlaps a substantial portion of the absorbent means 23. The amount of overlap between the stabilizing element 50 and the absorbent means 23 is preferably in a range of from 1 mm to about 20 mm. In an alternative embodiment (not shown) the stabilizing element extends continuously from one union means 15 to the opposite union means 15' and thus provides stability throughout the entire length of the secondary absorbent member.

In accordance with an alternative embodiment, it has been found that by adhesively securing the transverse end regions of the secondary absorbent member to the user's undergarment in the region underlying the union means that sufficient stability is provided to the compound sanitary napkin that the ends do not tend to fold inward upon application of downward pressure to the primary absorbent member. Thus, as shown in FIGURES 2 and 5, the attachment means 40 may be substantially vertically aligned with at least a portion of the union means 15 to secure transverse end of the secondary absorbent member to the user's undergarment. The use of attachment means 40 to stabilize the transverse ends 36, 37 has been found to be particularly useful when the transverse end regions are very flexible such as when the secondary absorbent element 30 does not include an absorbent element and/or when the union means 15 is located solely in the respective flanges 24, 34.

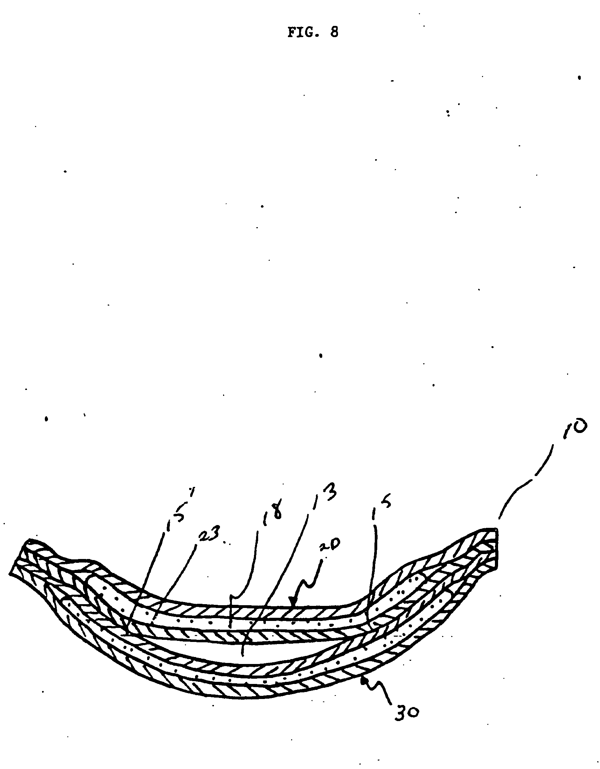

FIGURE 8 illustrates another embodiment of a compound sanitary napkin. In accordance with this embodiment, compound sanitary napkin 10 includes primary absorbent member 20 and secondary absorbent member 30. Primary absorbent member 20 is affixed to secondary absorbent member at union means 15, 15' at each transverse end of the napkin. Each union means 15, 15' extends from the transverse ends of the primary absorbent member 20 inward towards a center portion 18. Each union means 15, 15' is in vertical registration with at least a portion of the absorbent means 23. In accordance with this embodiment, the unattached center region 13 of the compound sanitary napkin 10 extends about one third of the length of the napkin, while each respective union means 15, 15' extends about one third of the length of the napkin. This embodiment has been found to provide enhanced body fit while minimizing the amount of lateral movement of the primary absorbent member 20 relative to the secondary absorbent member 30.

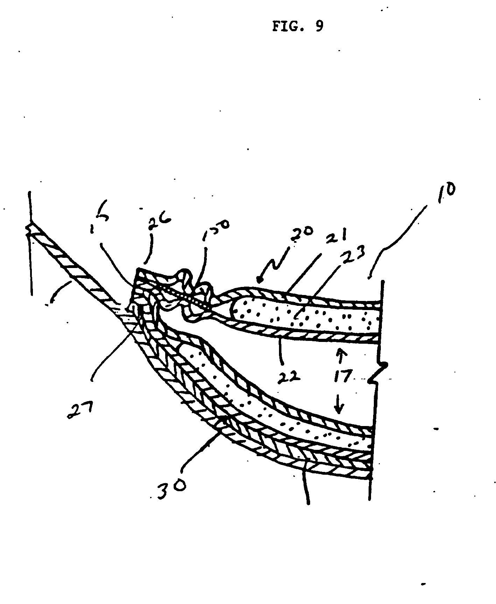

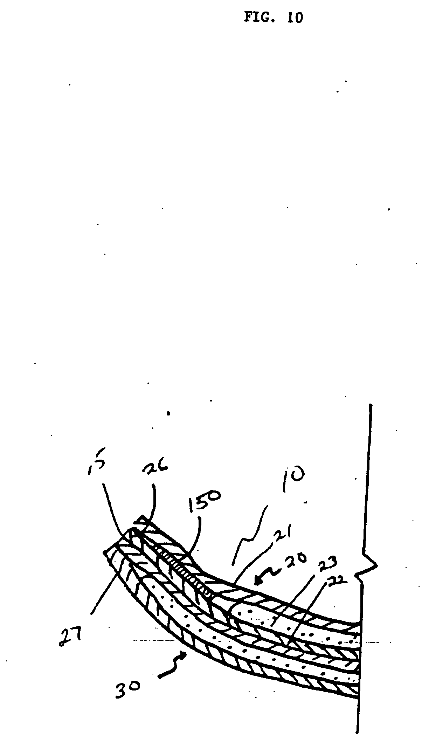

FIGURES 9 and 10 illustrate another embodiment of a compound sanitary napkin. In accordance with this embodiment, at least one transverse end and optionally both transverse ends 26, 27 of the primary absorbent member 20 are provided with an elastic element 150. The elastic element 150 is affixed to the topsheet 21, to the backsheet 22 or to both the topsheet 21 and the backsheet 22 in a pre-tensioned condition. The elastic element 150 may be affixed continuously to one or both of these layers or alternatively may be affixed at discrete, spaced apart locations. In a preferred embodiment, the elastic element 150 is located intermediate the topsheet 21 and the backsheet 22 and is adhesively affixed to both layers at discrete locations that are spaced apart along a longitudinal direction. The elastic element 150 may be applied to the compound napkin by stretching the elastic element 150 beyond its normal length and then affixing it to the above-described components of the primary absorbent member 20. The elastic element 150 is preferably resilient and capable of substantially returning to its original pre-stretched length upon removal of any external forces. The elastic element is then allowed to resume its original length in a relaxed condition as shown in FIG 9. This effectively reduces the length of the center portion 18 of the primary absorbent member 20 and creates the gap 17. Thus, the topsheet 21 and backsheet 22 are pulled inward toward the lateral centerline of the napkin and form a wrinkled appearance. FIG 10 shows the compound sanitary napkin in use when subjected to downward compressive forces. These compressive forces that are imparted to the napkin cause the elastic element 150 to stretch, thereby increasing the length of the center portion 18 and permitting the primary absorbent member 20 to contact the secondary absorbent member 30 along a substantial portion of their respective lengths. Thus, the elastic element 150 provides the napkin with enhanced resiliency to maintain close body fit in a comfortable manner.

The elastic element 150 may comprise a single strip of elastic material or alternatively may include a plurality of thin elastic strips that are individually placed in tension in the transverse ends of the napkin. In accordance with this embodiment, the elastic strips may extend continuously from the first transverse end 26 to the opposite second transverse end 17. The elastic strips are preferably located adjacent the longitudinal side edges of the primary absorbent member 20. The elastic strips are preferably located in a peripheral flange formed along the longitudinal sides by affixing the cover and barrier layers together. Any conventional elastic materials that are utilized in disposable absorbent products are suitable for use as an elastic element 150.

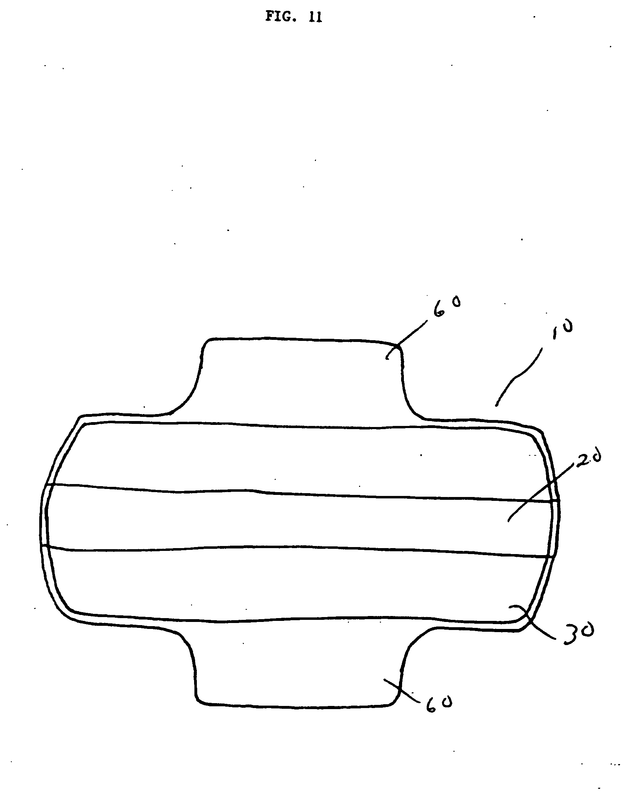

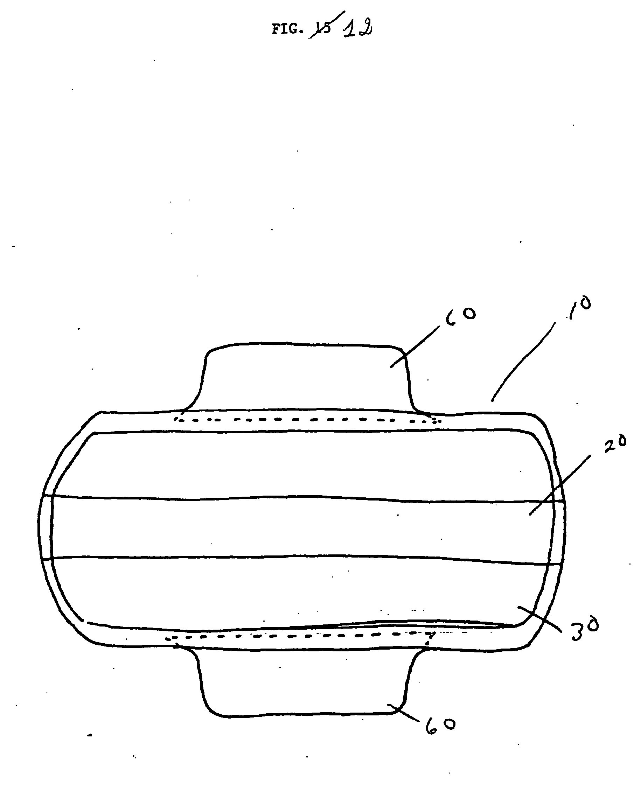

As shown in FIGURES 11 and 12, the secondary absorbent member 30 may have two side flaps 60. In accordance with FIG 11, each flap 60 is adjacent to and extends laterally from the side edge of the absorbent core. In accordance with FIG 12, each flap is affixed to the barrier sheet along a line of juncture that is inward from the longitudinally extending sides 25, 25'. In either embodiment, the flaps 60 are flexible and configured to be folded over the edges of the wearer's panties in the crotch region so that the flaps 60 are disposed between the edges of the wearer's panties and the wearer's thighs. The flaps 60 serve at least two purposes, to prevent soiling of the wearer's body and panties by menstrual fluid and to adhesively attach the napkin to an undergarment in use. In this way, the flaps 60 serve to keep the sanitary napkin properly positioned in the undergarment.

In a preferred embodiment, the flaps 60 are comprised of the topsheet and barrier sheet. Further, the flaps 60 are preferably unitary to the laminae of the secondary absorbent element wherein portions of the topsheet and barrier sheet extend laterally outward from the edges of the absorbent core in a central region of the sanitary napkin to form flexible flaps 60. However, the flaps 60 need not be unitary with the secondary absorbent member, but can be separate elements that are joined to the secondary absorbent member. Further, the flaps 60 can be comprised of a single substrate or other laminate configurations. It is preferred, however, that the flaps 60 have a liquid impervious barrier sheet to prevent exudates that reach the flaps 60 from soiling the edges of the wearer's panties.

Further, the flaps 60 may optionally be provided with an absorbent member, at least to a point beyond the edges of the wearer's panties. Theoretically, only a relatively small amount of menses should reach the flaps 60, therefore, only a relatively small amount of absorbent material is desirable in the flaps 60. However, at least some absorbent material is recommended in order to prevent any exudates that reach the flaps 60 from being able to flow further to unprotected areas. The absorbent material may be a tissue, or an extension of the absorbent element . However, the absorbent material in the flaps 60 should be relatively highly flexible.

A number of sanitary napkins having flaps 60 suitable or adaptable for use with the secondary absorbent member 20 of the compound sanitary napkin 40 of the present invention are disclosed in U.S. Pat. No. 4,687,478 issued to Van Tilburg on Aug. 18, 1987; U.S. Pat. No. 4,589,876 issued to Van Tilburg on May 20, 1986; and U.S. Pat No. 4,608,047 issued to Mattingly on Aug. 26, 1986.

The individual components of the primary absorbent member 20 and the secondary absorbent member 30 may be comprised of components that are extensible (preferably, capable of stretching) particularly in the longitudinal direction when the compound sanitary napkin is worn. Suitable components include, but are not limited to elastic films, elastic non-woven fabrics, visco-elastic polymeric materials, and the like and combinations thereof. Preferably, the primary absorbent member, particularly a transverse end portion thereof, is capable of elongating in the longitudinal direction between about 15% and about 40% of its unstretched length. This extensibility provides better in-use fit, comfort, and decreased staining when the compound sanitary napkin is affixed to the wearer's undergarments.

The secondary absorbent member may also include the above stretchable components and are preferably formed from polymeric mateials having visco-elastic properties that are extensible when the compound sanitary napkin is worn. Preferably, the compound sanitary napkin is capable of elongating between about 15% and about 40% of its unstretched original length. The topsheet for both the primary absorbent member and the secondary absorbent member may comprise an elastic, three-dimensional, fluid pervious, polymeric web.

## Claims

1. A compound sanitary napkin (10) adapted to be worn in a wearer's undergarment comprising;
- an uppermost primary absorbent member (20) having an absorbent core (23) and a fluid pervious topsheet (21) superposed on the absorbent core (23), a first transverse end (26) and an opposite second transverse end (27) and a first longitudinal side (25) and an opposite second longitudinal side (25) defining therebetween a width;
- a lowermost secondary absorbent member (30) having a fluid pervious topsheet (31) and a fluid impervious barrier sheet (32) joined to at least a portion of the topsheet (31), a first transverse end (36) and an opposite second transverse end (37) and a first longitudinal side (35) and an opposite second longitudinal side (35') defining therebetween a width,
- at least a portion of the primary absorbent member (20) along or adjacent to its first transverse end (26) is joined to at least a portion of the secondary absorbent member (30) along or adjacent to its first transverse end (36) at a first union means (15);
- at least a portion of the primary absorbent member (20) along or adjacent to its second transverse end (27) is joined to at least a portion of the secondary absorbent member (30) adjacent along or adjacent to its second transverse end (37) at a second union means (15');
- the primary absorbent member (20) having a center portion (18) intermediate the first and second union means (15, 15');
- the secondary absorbent member (30) having a central region (39) intermediate the first and second union means (15, 15');
- the center portion (18) and the central region (39) being unaffixed to each other and each having a respective length measured from the first union means (15) to the second union means (15'), wherein the center portion (18) of the primary absorbent member (20) has a length that is less than the length of the central region (39) of the secondary absorbent member (30) and the length of the central region (39) of the secondary absorbent member (30) is foreshortened and wherein at least a portion of the center portion (18) of the primary absorbent member (20) is spaced apart from the central region (39) of the secondary absorbent member (30) to provide a dynamic fit to the wearer, **characterized in that**
the secondary absorbent member (30) has a stabilising element (50) in at least one transverse end (36, 37), the stabilising element (50), when subjected to an inwardly compressive force, is adapted to provide a bending moment in the secondary absorbent member (30) that is located at a position that is spaced apart from the union means (15, 15').

2. The compound sanitary napkin (10) according to claim 1, wherein the stabilizing member (50) includes an additional adhesive layer that is capable of imparting stiffness to the transverse end of the secondary absorbent member (30).

3. The compound sanitary napkin (10) according to claim 1, wherein the stabilizing element (50) has a first end that is in vertical registration with at least a portion of the union means (15) and extends longitudinally inward towards the center region to an extent that a second opposite end of the stabilizing element (50) is in vertical registration with at least a portion of the absorbent means (23) in the primary absorbent member (20).

4. The compound sanitary napkin (10) according to claim 1, wherein the amount of overlap between the stabilizing element (50) and the absorbent means (23) is in a range of from 1mm to 20 mm.

## Patentansprüche

1. Verbund-Damenbinde (10), die dazu ausgelegt ist, in der Unterwäsche einer Trägerin getragen zu werden, mit:
- einem obersten primären absorbierenden Element (20) mit einem absorbierenden Kern (23) und einer fluiddurchlässigen Oberlage (21), die über dem absorbierenden Kern (23) liegt, einem ersten Querende (26) und einem gegenüberliegenden zweiten Querende (27) und einer ersten Längsseite (25) und einer gegenüberliegenden zweiten Längsseite (25), die zwischen sich eine Breite definieren;
- einem untersten absorbierenden Element (30) mit einer fluiddurchlässigen Oberlage (31) und einer fluidundurchlässigen Barrierelage (32), die mit wenigstens einem Teil der Oberlage (31) verbunden ist, einem ersten Querende (36) und einem gegenüberliegenden zweiten Querende (37) und einer ersten Längsseite (35) und einer gegenüberliegenden zweiten Längsseite (35'), die zwischen sich eine Breite definieren;
- wobei wenigstens ein Teil des primären absorbierenden Elementes (20) entlang oder benachbart seinem ersten Querende (26) mit wenigstens einem Teil des sekundären absorbierenden Elementes (30) entlang oder benachbart seinem ersten Querende (36) bei einer ersten Verbindungseinrichtung (15) verbunden ist;
- wobei wenigstens ein Teil des primären absorbierenden Elementes (20) entlang oder benachbart seinem zweiten Querende (27) mit wenigstens einem Teil des sekundären Element (30) entlang oder benachbart seinem zweiten Querende (27) bei einer zweiten Verbindungseinrichtung (15') verbunden ist;
- wobei das primäre absorbierende Element (20) einen mittleren Teil (18) zwischen der ersten und zweiten Verbindungseinrichtung (15, 15') hat;
- wobei das sekundäre absorbierende Element (30) einen mittleren Bereich (39) zwischen der ersten und zweiten Verbindungseinrichtung (15, 15') hat;
- wobei der mittlere Teil (18) und der mittlere Bereich (39) aneinander unbefestigt sind und jeder eine jeweilige Länge hat, die von der ersten Verbindungseinrichtung (15) zu der zweiten Verbindungseinrichtung (15') gemessen wird, wobei der mittlere Teil (18) des primären absorbierenden Elementes (20) eine Länge hat, die geringer ist als die Länge des mittleren Bereiches (39) des sekundären absorbierenden Elementes (30) und die Länge des mittleren Bereiches (39) des sekundären absorbierenden Elementes (30) vorverkürzt ist und wobei wenigstens ein Teil des mittleren Teiles (18) des primären absorbierenden Elementes (20) von dem mittleren Bereich (39) des sekundären absorbierenden Elementes (30) beabstandet ist, um eine dynamische Anpassung an den Träger zur Verfügung zu stellen, **dadurch gekennzeichnet, daß**
das sekundäre absorbierende Element (30) ein stabilisierendes Element (50) in wenigstens einem Querende (36, 37) hat, wobei das stabilisierende Element (50), wenn es einer nach innen gerichteten Kompressivkraft ausgesetzt ist, so ausgelegt ist, daß es in dem sekundären absorbierenden Element (20) ein Biegemoment bildet, das sich an einem Ort befindet, der von der Verbindungseinrichtung (15, 15') beabstandet ist.

2. Verbund-Damenbinde (10) nach Anspruch 1, bei der das stabilisierende Element (50) eine zusätzliche Klebemittelschicht umfaßt, die in der Lage ist, das Querende des sekundären absorbierenden Elementes (30) mit Steifigkeit auszustatten.

3. Verbund-Damenbinde (10) nach Anspruch 1, bei der das stabilisierende Element (50) ein erstes Ende hat, das in vertikaler Ausrichtung mit wenigstens einem Teil der Verbindungseinrichtung (15) ist und sich in Längsrichtung nach innen auf den mittleren Bereich in einem Ausmaß zu erstreckt, daß ein zweites gegenüberliegendes Ende des stabilisierenden Elementes (50) in vertikaler Ausrichtung mit wenigstens einem Teil der absorbierenden Einrichtung (23) in dem primären absorbierenden Element (20) ist.

4. Verbund-Damenbinde (10) nach Anspruch 1, bei der das Ausmaß der Überlappung zwischen dem stabilisierenden Element (50) und der absorbierenden Einrichtung (23) in einem Bereich von 1 mm bis 20 mm liegt.

## Revendications

1. Serviette hygiénique composée (10) conçue pour être portée dans un sous-vêtement de la personne comprenant :
un élément absorbant primaire supérieur (20) ayant un noyau absorbant (23) et une feuille supérieure perméable au fluide (21) superposée au noyau absorbant (23), une première extrémité transversale (26) et une seconde extrémité transversale opposée (27) et un premier côté longitudinal et un second côté longitudinal opposé (25) définissant entre ceux-ci une largeur ;
un élément absorbant secondaire inférieur (30) ayant une feuille supérieure perméable au fluide (31) et une feuille barrière imperméable au fluide (32) reliées à au moins une portion de la feuille supérieure (31), une première extrémité transversale (36) et une seconde extrémité transversale opposée (37) et un premier côté longitudinal (35) et un second côté longitudinal opposé (35') définissant entre ceux-ci une largeur ;
au moins une portion de l'élément absorbant primaire (20) disposée le long de ou de manière adjacente à la première extrémité transversale (26) est reliée à au moins une portion de l'élément absorbant secondaire (30) disposée le long de ou de manière adjacente à la première extrémité transversale (36) au niveau d'un premier moyen de liaison (15) ;
au moins une portion de l'élément absorbant primaire (20) disposée le long de ou de manière adjacente à la seconde extrémité transversale (27) est reliée à au moins une portion de l'élément absorbant secondaire (30) disposée le long de ou de manière adjacente à la seconde extrémité transversale (37) au niveau d'un second moyen de liaison (15') ;
l'élément absorbant primaire (20) ayant une portion centrale (18) intermédiaire entre le premier et le second moyens de liaison (15, 15') ;
l'élément absorbant secondaire (30) ayant une région centrale (39) intermédiaire entre le premier et le second moyens de liaison (15, 15') ;
la portion centrale (18) et la région centrale (39) n'étant pas fixées l'une à l'autre et chacune ayant une longueur respective mesurée à partir du premier moyen de liaison (15) jusqu'au second moyen de liaison (15'), dans laquelle la portion centrale (18) de l'élément absorbant primaire (20) présente une longueur qui est inférieure à la longueur de la région centrale (39) de l'élément absorbant secondaire (30) et la longueur de la région centrale (39) de l'élément absorbant secondaire (30) est raccourcie, et dans laquelle au moins une portion de la portion centrale (18) de l'élément absorbant primaire (20) est espacée de la région centrale (39) de l'élément absorbant secondaire (30) pour être adapté de manière dynamique à la personne, **caractérisée en ce que**
l'élément absorbant secondaire (30) présente un élément de stabilisation (50) dans au moins une extrémité transversale (36, 37), l'élément de stabilisation (50), lorsqu'il est soumis à une force de compression dirigée vers l'intérieur, est conçu pour produire un moment de flexion dans l'élément absorbant secondaire (30) qui est situé en une position qui est espacée des moyens de liaison (15, 15').

2. Serviette hygiénique composée (10) selon la revendication 1, dans laquelle l'élément de stabilisation (50) comprend une couche adhésive supplémentaire qui est capable de doter l'extrémité transversale de l'élément absorbant secondaire (30) d'une rigidité.

3. Serviette hygiénique composée (10) selon la revendication 1, dans laquelle l'élément de stabilisation (50) présente une première extrémité qui est dans une position verticale avec au moins une portion du moyen de liaison (15) et s'étend longitudinalement vers l'intérieur vers la région centrale jusqu'à une seconde extrémité opposée de l'élément de stabilisation (50) qui est dans une position verticale avec au moins une portion des moyens absorbants (23) dans l'élément absorbant primaire (20).

4. Serviette hygiénique composée (10) selon la revendication 1, dans laquelle la quantité de chevauchement entre l'élément de stabilisation (50) et les moyens absorbants (23) est dans un intervalle de 1 mm à 20 mm.
